# EUROPEAN PATENT SPECIFICATION

(11) **EP 4 575 862 B1**
(45) Date of publication and mention of the grant of the patent: **10.12.2025**
(21) Application number: 24221358.5
(22) Date of filing: 19.12.2024
(51) Int. Cl.: H04L 67/12, G16H 40/40, G06F 21/57, G06F 8/65, G16H 40/67

(54) **DECENTRALIZED SECURE DISTRIBUTION OF SOFTWARE UPDATES TO MEDICAL DEVICES**
DEZENTRALISIERTE SICHERE VERTEILUNG VON SOFTWAREAKTUALISIERUNGEN AN MEDIZINISCHE VORRICHTUNGEN
DISTRIBUTION SÉCURISÉE DÉCENTRALISÉE DE MISES À JOUR LOGICIELLES À DES DISPOSITIFS MÉDICAUX

(30) Priority: 20.12.2023 US 202363612525 P; 11.12.2024 US 202418976519
(43) Date of publication of application: 25.06.2025
(73) Proprietor: Biosense Webster (Israel) Ltd., 2066717 Yokneam (IL)
(72) Inventor: RAVUNA, Eliyahu, 2066717 Yokneam (IL); YAACOBOVICH, Or, 2066717 Yokneam (IL)
(74) Representative: Carpmaels & Ransford LLP

(56) References cited:
- US-A1- 2020 151 335
- US-A1- 2022 270 754
- KEN FUCHS: "IEEE P2933/D3", vol. 2733, 23 October 2023 (2023-10-23), pages 1 - 250, XP068295793, Retrieved from the Internet <URL:https://ieee-sa.imeetcentral.com/p/aQAAAAAFEq53> [retrieved on 20231030]

## Description

### RELATED APPLICATION INFORMATION

The present application claims benefit of US Provisional Patent Application S/N 63/612,525 of Ravuna, et al., and entitled "Decentralized secure distribution of software updates to medical devices" filed 20 December 2023.

### FIELD OF THE INVENTION

The present disclosure relates to medical systems, and in particular, but not exclusively, to distribution of software updates to medical devices.

### BACKGROUND

Software updates are crucial to digital safety and cybersecurity, and provide other benefits, including repairing security holes, fixing computer bugs, and adding new features to devices. Hackers may take advantage of software vulnerabilities, for example by writing code to target the vulnerability.

Updating software and operating systems helps keep hackers out, protects the data, and ensures that the software is performing according to latest operational methods, which is particularly important for sensitive data and medical devices running life critical, or other, applications.

US2022/270754A1 discloses a mesh-based medical device network for relaying software updates between devices. US2020/151335A1 discloses the use of digital signatures for verifying the authenticity and integrity of firmware updates. IEEE P2933/D3, "Draft Standard for Clinical Internet of Things (IoT) Data and Device with TIPPSS," vol. 2733 23 October 2023, pages 1-250, XP068295793 discloses general security requirements and best practices for OTA updates in clinical IoT systems.

### BRIEF DESCRIPTION OF THE DRAWINGS

The present disclosure will be understood from the following detailed description, taken in conjunction with the drawings in which:
Fig. 1 is a block diagram view of a computer system in a medical facility constructed and operative in accordance with an exemplary mode of the present disclosure;
Fig. 2 is a block diagram view of another computer system in a medical facility constructed and operative in accordance with another exemplary mode of the present disclosure;
Fig. 3 is a block diagram view of another computer system in a medical facility constructed and operative in accordance with yet another exemplary mode of the present disclosure;
Fig. 4 is a block diagram view of a medical device in the system of Fig. 1;
Fig. 5 is a block diagram of a memory and stored tables in the device of Fig. 4;
Figs. 6-9 are views of routing tables illustrating generation of routing tables for use in the system of Fig. 1;
Fig. 10 is a flowchart including steps in a method of generating routing tables for use in the system of Fig. 1;
Fig. 11 is a flowchart including steps in a method of providing software updates to the device of Fig. 4;
Fig. 12 is a flowchart including steps in a method of receiving and processing files in the device of Fig. 4;
Fig. 13 is a flowchart including steps in a method of receiving and processing logs in the device of Fig. 4; and
Fig. 14 is a flowchart including steps in a method of receiving and processing logs received by a cloud agent in the system of Fig. 1.

### DESCRIPTION OF EXAMPLE EXEMPLARY MODES

### OVERVIEW

As previously mentioned, it is critical for device software to be updated as needed to maintain safety, security, and effectiveness throughout the product's lifecycle. This is particularly important for medical devices. According to "Cybersecurity in Medical Devices: Quality System Considerations and Content of Premarket Submissions" issued by FDA on September 27, 2023, timely updatability and patchability is one of the security objectives assessed by FDA when examining medical devices.

Many medical devices in a medical facility, such as a hospital, are not directly connected to the Internet for security reasons and/or based on lack of device capabilities, for example. Therefore, such devices do not receive the relevant cybersecurity and other updates in a timely manner, if at all. This is especially critical for Cybersecurity updates because these updates need to be distributed quickly. The delay in distributing cybersecurity updates increases the risk of a zero-day attack.

Therefore, exemplary modes of the present disclosure address some of these drawbacks by providing a system in which medical devices installed in a medical facility have agents which facilitate the transfer of software updates from one or more cloud software update agents to the medical devices. Even though not all of the medical devices are connected to the Internet, one or more of the medical devices may be connected to the Internet to receive the software updates from the cloud software update agent(s) on behalf of the medical devices which are not directly connected to the Internet. The medical devices which are not directly connected to the Internet are nevertheless connected (directly or indirectly) to the medical device(s) which is/(are) connected to the Internet via a suitable communication link such as a USB connection and/or an Ethernet connection, for example. The received software updates may then be distributed via the network of agents installed in the medical devices in the medical facility. In such a manner, software updates may be forwarded to all the connected medical devices in the medical facility to ensure that each device has updated medical software. The cloud software agent(s) may provide software updates for different manufacturers of the medical devices installed in the medical facility. In some exemplary modes, the cloud software agents may include an agent for each manufacturer of the medical devices installed in the medical facility. For example, a cloud software agent for manufacturer A may be connected to a medical device of manufacturer A in the medical facility, and a cloud software agent for manufacturer B may be connected to a medical device of manufacturer B in the medical facility.

The distribution of software updates may include a zero-trust method in which the medical devices installed in the medical facility do not need to trust each other which is especially important when the medical devices are produced by different manufacturers. The above method may also use existing data connections between the devices.

The agents installed in the medical devices perform a discovery method to discover the medical devices installed in the medical facility and the cloud agent(s) and the topology of the connections between the medical devices and the cloud agent(s), as described in disclosed exemplary modes. The discovery method yields routing tables installed in each of the medical devices and the cloud agent(s) and allows files such as software updates to be forwarded from the cloud agent(s) to relevant medical devices, and for software update logs and other log files to be forwarded by the medical devices to the cloud agent(s). The discovery method may be applied to basic topologies or more complex mesh topologies, for example, including loops, as described in disclosed exemplary modes.

An agent operating in a medical device may provide a received software update to be installed in that medical device, and forward other received software updates to other medical devices according to the destination addresses for the corresponding received software updates. An agent operating in a medical device may receive logs from that medical device or from other medical devices for forwarding to the cloud agent(s) via one or more medical devices installed in the medical facility. In some cases, when the medical device is directly connected to the Internet, that medical device may forward the logs to the cloud agent(s) via the Internet connection.

Each agent may maintain and store a file delivery table (providing indications of files received for delivery such as file names, and corresponding destination information such as model and serial numbers), a routing table (providing identification information of assessable medical devices such as model and serial numbers, and corresponding routing information such as number of hops to the medical devices and via which neighbor(s)), and a direct neighbor table (providing a list of direct neighbors such as model and serial numbers, and corresponding address information such as IP address(es) of the direct neighbor(s)).

The system may include various security features, described in more detail below.

In some exemplary modes, files such as software updates are signed by the manufacturer of the target medical device, for example, using a private key or certificate of the manufacturer. The signatures may be checked by the medical devices in which the software updates are to be installed, for example, using the public keys or certificates of the relevant manufacturers. The files may optionally be encrypted, for example, using the public keys of the corresponding medical devices for decryption with the private keys of the corresponding medical devices.

In some exemplary modes, one or more (e.g., all) agents which are not installed in the target device of a software update may also check the signature of the software update to prevent the network being flooded with unnecessary communications. If a medical device receives a file with a bad signature, and a neighboring device was meant to check that signature, then it may be assumed that the neighbor did not do a good job, for example, because the neighbor may be infected or compromised or there is a hacker imitating the neighbor. In this case, the medical device may automatically block this neighbor, e.g., for a predetermined time period.

In some exemplary modes, if an infected medical device refuses to relay files, the cloud agent may identify this behavior based on the target medical device not acknowledging installation of a software update and/or analyzing received log files to determine which medical device is not relaying the files. The operator may then block the medical device not relaying files (e.g., remove it from the routing tables) so that files automatically arrive at the destination without passing via the blocked medical device, if there is another available path.

A log may be signed (e.g., with the private key of the medical device producing the log) and authenticated in the cloud using a corresponding public key. A tamper resistant chip, e.g., trusted platform model (TMP) according to ISO/IEC 11889 standard, may be used to store the private key of the medical device. The log may optionally be encrypted using the manufacture public key for decrypting by the cloud agent.

In some exemplary modes, the agents may limit the size of files allowed to be received in order to prevent an attacker trying to send a giant file to deplete disk space as signatures are generally checked at the end of transmission. In some exemplary modes, files pending delivery in a medical device may be purged from a queue of files to be delivered if the files have been in the queue longer than a given time limit (e.g., number of days). In some exemplary modes, files pending delivery in a medical device may be purged from the queue, e.g., on a first-in-first-out (FIFO) basis if a maximum queue size is reached.

In some exemplary modes, if a medical device runs out of disk space, the medical device may proactively send a message to its neighbors to ask to be removed from the paths in the routing tables. In some exemplary modes, if a medical device is sending too many files in a given time period, this medical device may be marked as suspect and may be blocked for a specific amount of time.

### SYSTEM DESCRIPTION

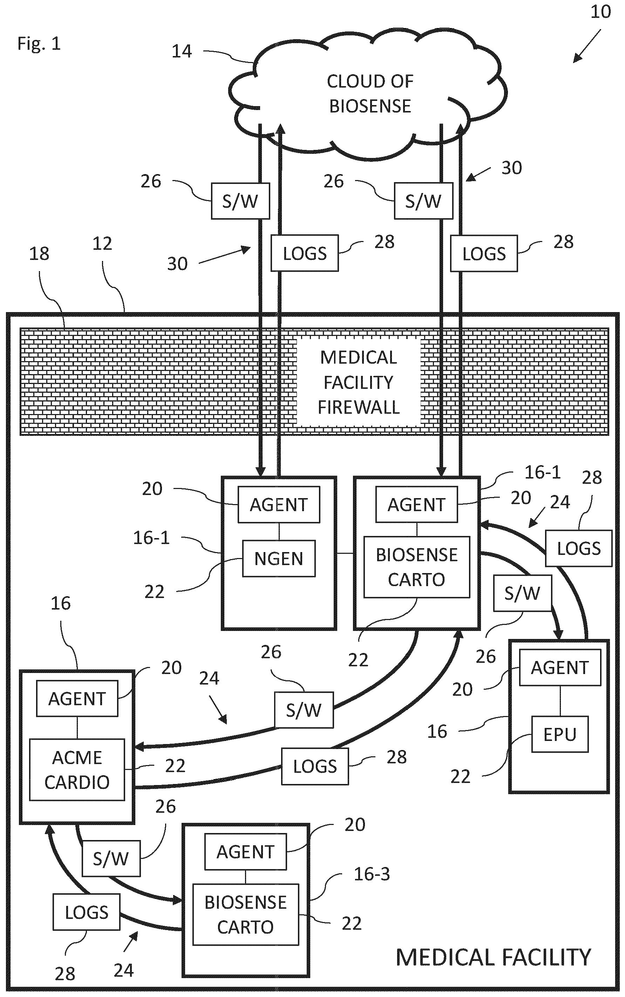

Reference is now made to Fig. 1, which is a block diagram view of a computer system 10 in a medical facility 12 constructed and operative in accordance with an exemplary mode of the present disclosure. The system 10 includes a cloud medical device software update agent 14 and medical devices 16. The medical devices 16 are installed in the medical facility 12. The medical facility 12 includes a firewall 18 to provide protection to the medical devices 16 from external traffic. Each medical device 16 includes a medical device software update agent 20 and medical device software 22. The medical devices 16 in the medical facility 12 form a network and are connected via any suitable data connections 24, for example, USB connections and/or Ethernet connections, or any suitable combination thereof. One or more of the medical devices 16 (e.g., medical devices 16-1) is configured to establish one or more corresponding Internet connections 30 with the cloud medical device software update agent 14. The cloud medical device software update agent 14 is configured to send software updates 26 to the medical devices 16 and receive logs 28 from the medical devices 16, described in more detail below. The cloud medical device software update agent 14 may include the server(s) of the medical device manufacturer in the cloud. For example, the cloud medical device software update agent 14 may be implemented using an Azure^{™} IoT System of Microsoft or a ThingWorx^{™} Server of PTC.

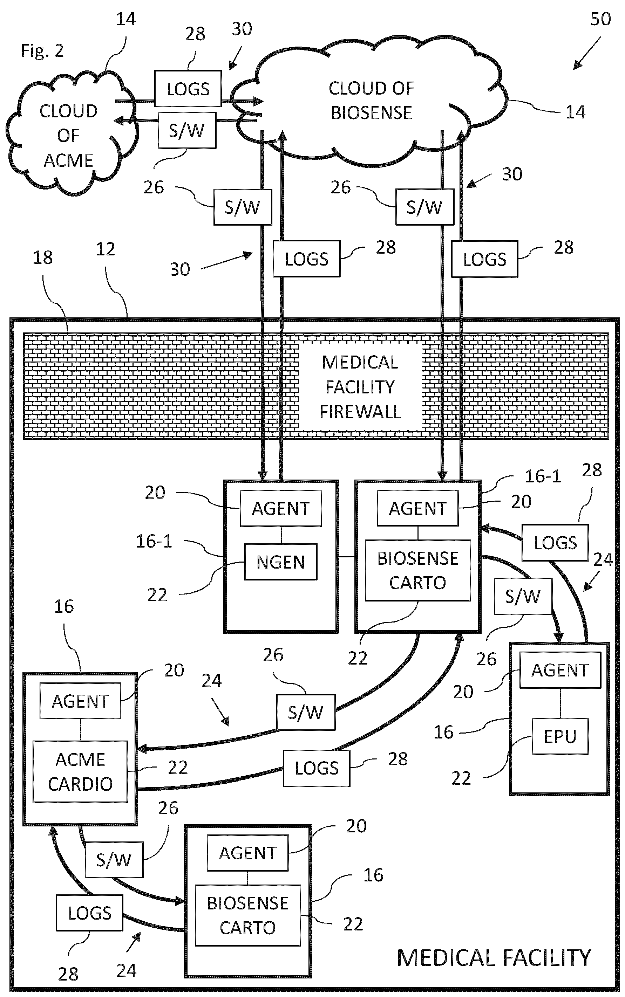

Reference is now made to Fig. 2, which is a block diagram view of another computer system 50 in the medical facility 12 constructed and operative in accordance with another exemplary mode of the present disclosure. The computer system 50 is substantially the same as system 10 except for the following differences. Computer system 50 includes two cloud medical device software update agents 14 for two corresponding manufacturers. In the example of Fig. 2, one of the cloud medical device software update agents 14 is for ACME, Inc. and one of the cloud medical device software update agents 14 is for Biosense Webster, Inc. The cloud medical device software update agents 14 are configured to be connected to each other via another Internet connection 30.

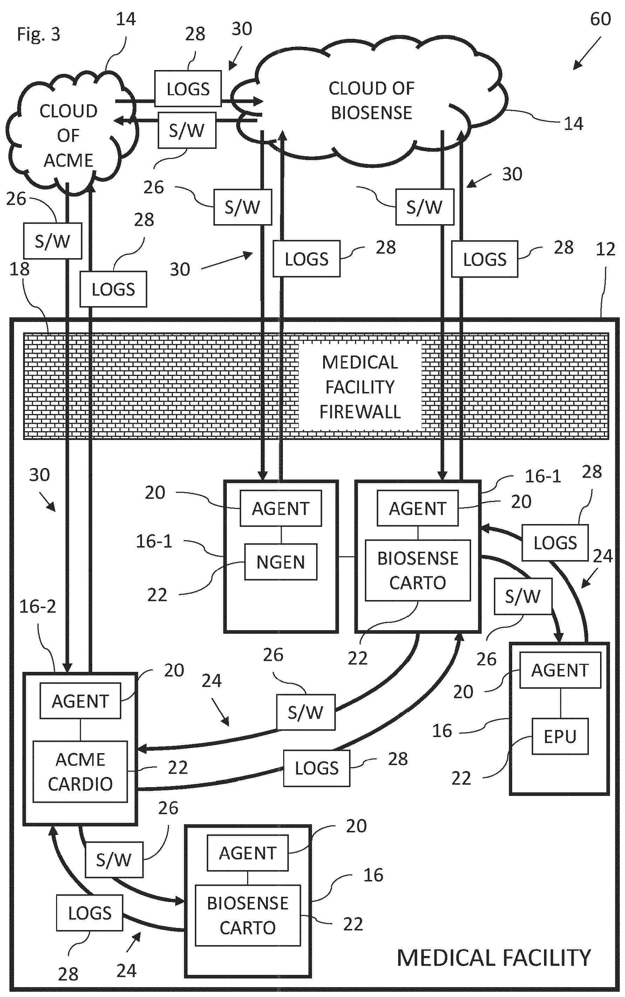

Reference is now made to Fig. 3, which is a block diagram view of another computer system 60 in the medical facility 12 constructed and operative in accordance with yet another exemplary mode of the present disclosure. The system 60 is substantially the same as computer system 50 except for the following differences. Both of the cloud medical device software update agents 14 are connected to at least one device 16 in the medical facility 12 via Internet connections 30. In the example of Fig. 3, the cloud medical device software update agent 14 of Biosense Webster, Inc. is configured to be connected to two medical devices 16-1 of Biosense Webster, Inc. via Internet connections 30, in the medical facility 12, and the cloud medical device software update agent 14 of ACME, Inc. is configured to be connected to the medical device 16-2 of ACME, Inc. via another one of the Internet connections 30.

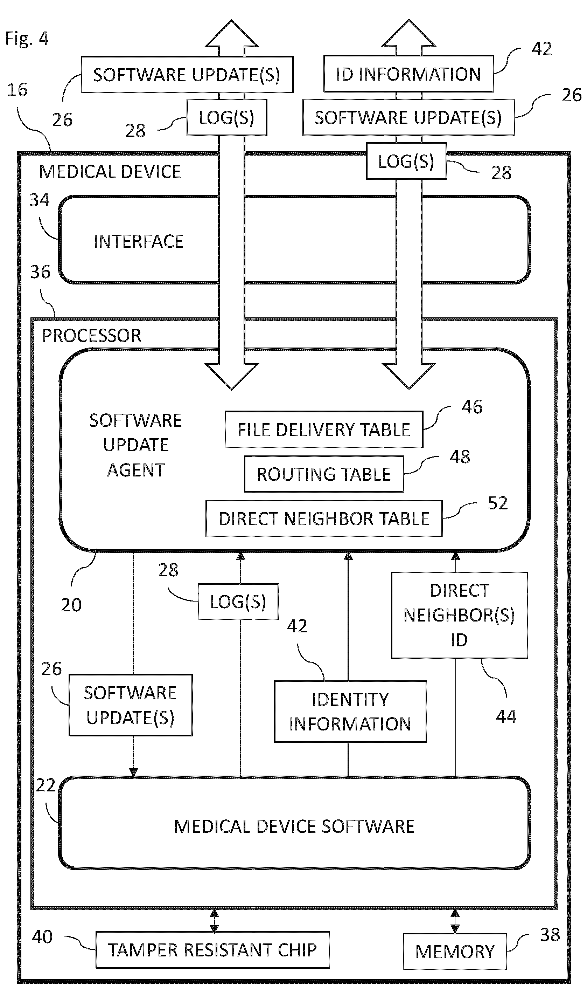

Reference is now made to Fig. 4, which is a block diagram view of one of the medical devices 16 (e.g., medical device 16-3) in the system 10 of Fig. 1. The medical device 16 includes an interface 34, a processor 36, a memory 38, and optionally a tamper resistant chip 40.

The interface 34 may be any suitable interface, for example, an Ethernet enabled interface, a USB enabled interface, and/or an Internet enabled interface. As previously mentioned, one or more of the medical devices 16 are directly connected to the Internet, while others of the medical devices 16 are not. One or more of the medical devices 16 may be non-internet enabled devices, which are not able to connect to the Internet by themselves or even process Internet Protocol (IP) packets, but able to share data over the Internet via a device which is connected to the Internet and can process IP packets. The interface 34 may be configured to share data with the cloud medical device software update agent 14 via the Internet using the internet connection of another medical device 16.

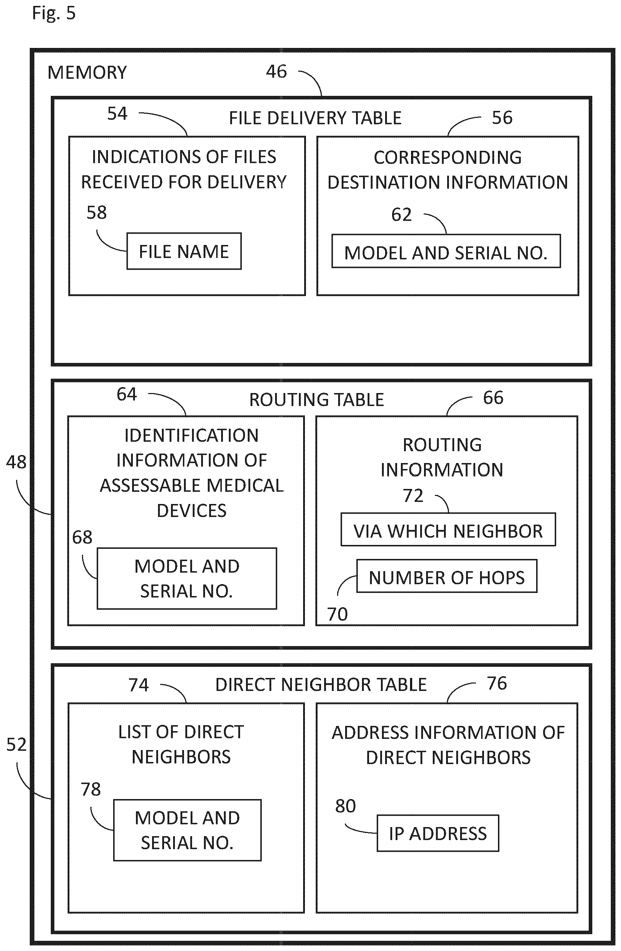

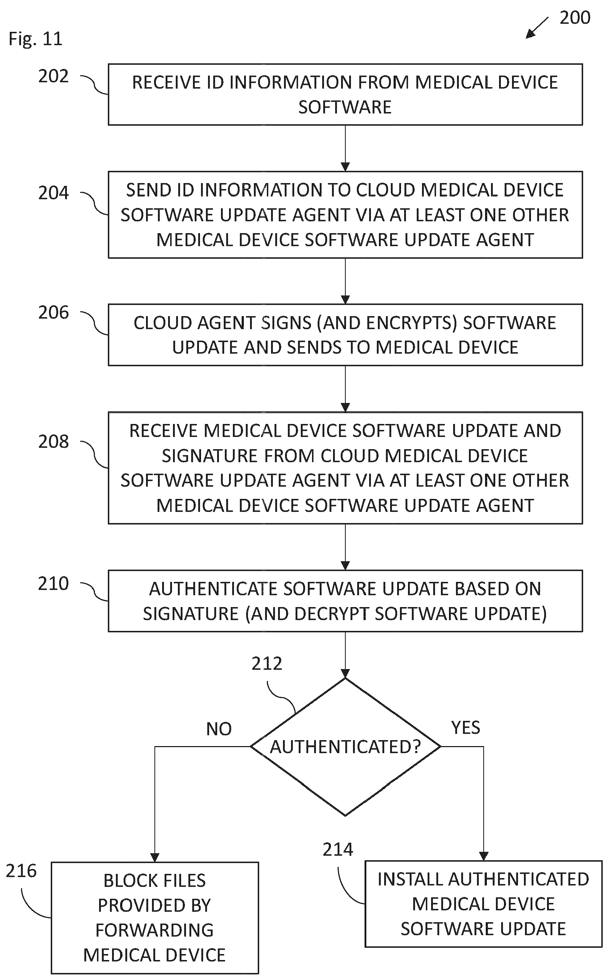

The processor 36 is configured to run agent 20 and medical device software 22. The agent 20 is configured to receive software updates 26 from cloud medical device software update agent 14 via the interface 34 optionally via one of more other medical devices 16. The received software updates 26 may include one or more software updates 26 for forwarding to other medical devices 16 via the interface 34. The agent 20 may also provide software updates 26 to update the medical device software 22. The medical device software 22 may provide logs 28 to the agent 20. The agent 20 may also receive logs 28 from agents 20 of other medical devices 16 via the interface 34. The agent 20 forwards the received logs to the cloud medical device software update agent 14, via the interface 34, and optionally via one or more medical devices 16. The medical device software 22 may provide the agent 20 with identity information 42 such as model and serial number for forwarding to the cloud medical device software update agent 14, as described in more detail with reference to Fig. 11. The medical device software 22 may provide the agent 20 with direct neighbor identification 44 as described in more detail below with reference to Fig. 5.

The agent 20 is configured to maintain tables, including a file delivery table 46, a routing table 48, and a direct neighbor table 52. The memory 38 is configured to stored tables 46, 48, 52, described in more detail below with reference to Fig. 5.

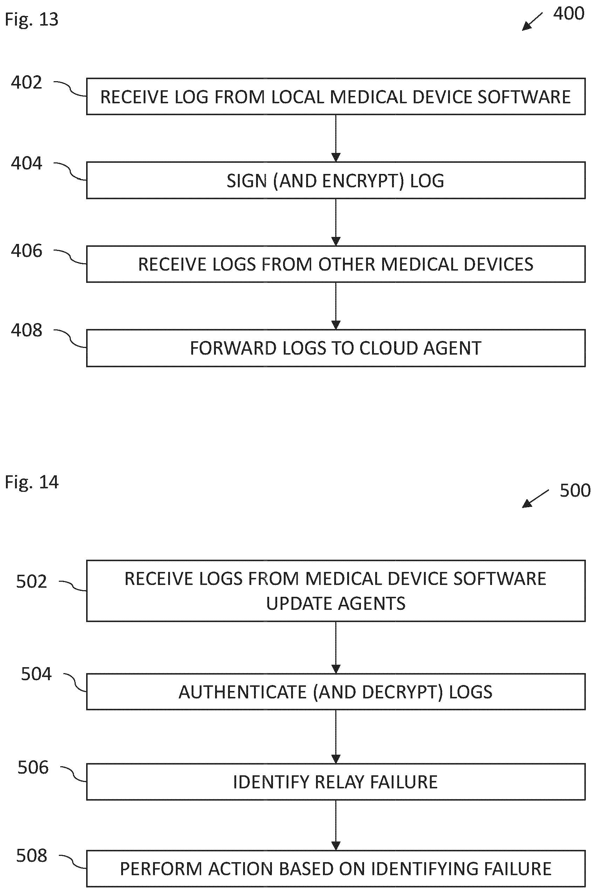

The tamper resistant chip 40 is configured to store a private key of the medical device 16. Each medical device 16 typically has its own unique private key. Use of the private key is described in more detail below in Figs. 11 and 13.

In practice, some, or all of the functions of the processor 36 may be combined in a single physical component or, alternatively, implemented using multiple physical components. These physical components may comprise hardwired or programmable devices, or a combination of the two. In some exemplary modes, at least some of the functions of the processor 36 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

Reference is now made to Fig. 5, which is a block diagram of memory 38 and stored tables in the device 16 of Fig. 4.

The file delivery table 46 includes indications 54 of one or more files received from cloud medical device software update agent 14 for delivery to one or more of the multiple medical devices 16 in the network, and corresponding destination information 56 of the file(s). The indications 54 may include a file name 58 and the destination information 56 may include a model and serial number 62 for each corresponding entry in the file delivery table 46.

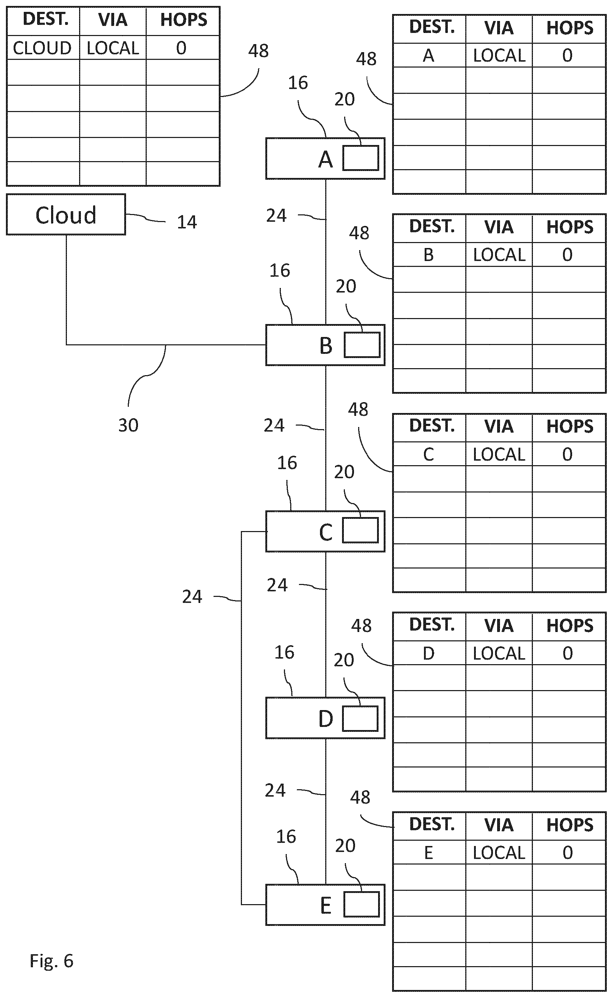

The routing table 48 includes identification information 64 of assessable ones of the medical devices 16 in the network, and corresponding routing information 66 of the assessable medical devices 16. The identification information 64 may include a model and serial number 68, and the routing information 66 may include a number of hops 70, and via which neighbor 72, for each entry in the table. The routing table 48 is described in more detail with reference to Figs. 6-10.

The direct neighbor table 52 includes a list 74 of one or more direct neighbors of the medical device 16 (in which the direct neighbor table 52 is stored), and address information 76 identifying the one or more direct neighbors in the network of medical devices 16. The list 74 may include a model and serial number 78 for each entry in the direct neighbor table 52. The address information 76 may include an IP address 80 for each corresponding entry in the table. A direct neighbor is one of the medical devices 16 connected directly to the medical device 16 in which the direct neighbor table 52 is stored, without an intervening medical device 16. The direct neighbor table 52 may be populated based on the direct neighbor identification(s) 44 (Fig. 4) received from the medical device software 22.

Reference is now made to Figs. 6-9, which are views of routing tables 48 illustrating generation of routing tables 48 for use in the system 10 of Fig. 1.

Figs. 6-9 show devices 16, labelled devices A to E for convenience, and a cloud agent 14. The cloud medical device software update agent 14 is connected to device B via Internet connection 30, device B is directly connected to devices A and C via data connections 24, device D is directly connected to device C and E via data connections 24, and device C is connected directly to device E with one of the data connections 24.

Fig. 6 shows that each agent 14, 20 has added its own device to its own routing table 48 listing the destination as the name of its own device, and set "via" to local, and hop value to zero.

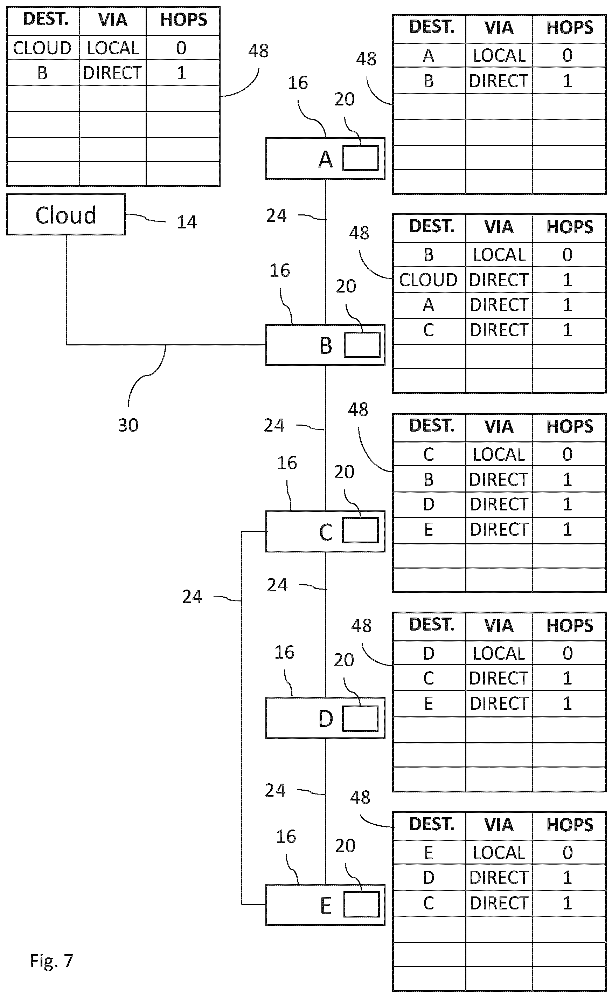

Fig. 7 shows that each agent 14, 20 has added its direct neighbors to its own routing table 48 based on the direct neighbor table 52 of each agent 14. 20. For each direct neighbor entry in the routing tables 48, the destination name of the direct neighbor is given (e.g., cloud, or A, or B etc.), "via" is set as "direct", and hop value is set to 1.

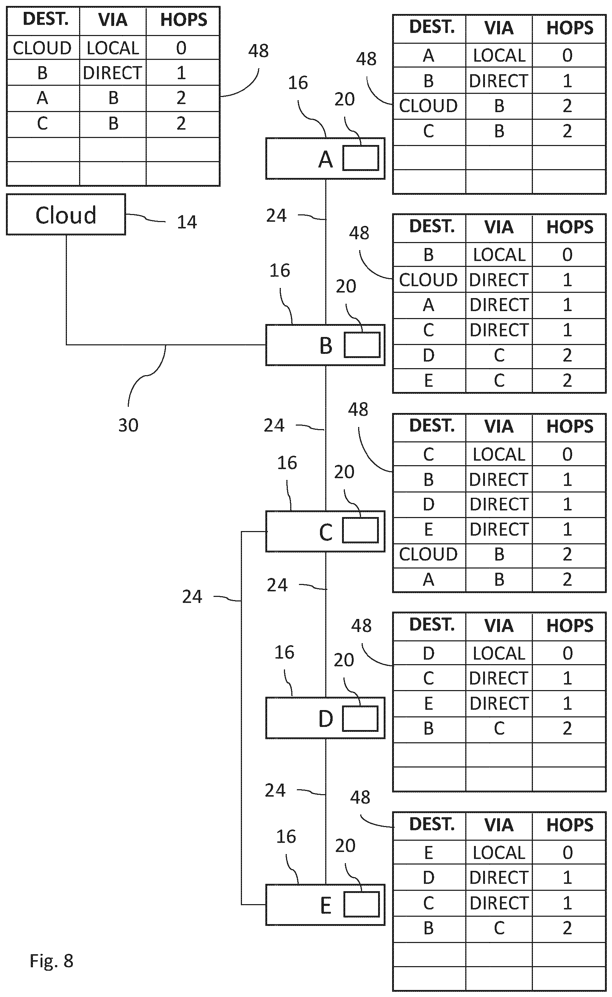

Fig. 8 shows the result of each agent 14, 20 providing its routing table 48 to its direct neighbor(s) and receiving the routing table(s) 48 from its direct neighbor(s). The received routing table(s) 48 is/(are) used by the receiving agent 14, 20 to update its own routing table 48, as now described in more detail.

Each entry in the received routing table(s) is updated so that each hop value is increased by 1, and the "via" is set to the identity of the entity sending the routing table. For example, if the routing table 48 is received from device A, "via" is set to device A. Additionally, duplicate entries are removed to leave the entry with the shortest number of hops, as illustrated in the example below.

For example, when device C receives the routing table 48 from device D, device C receives the following entries (as shown in Fig. 7):
D via local with hop of 0;
C via direct with hop of 1; and
E via direct with hop of 1.

The above entries are updated by the agent 20 of device C to:
D via D with hop of 1;
C via D with hop of 2; and
E via D with hop of 2.

Device C also receives the routing table 48 from device B with the following entries (as shown in Fig. 7):
B via local with hop of 0;
Cloud via direct with hop of 1;
A via direct with hop of 1; and
C via direct with hop of 1.

The above entries are updated by the agent 20 of device C to:
B via B with hop of 1;
Cloud via B with hop of 2;
A via B with hop of 2; and
C via B with hop of 2.

Device C also receives the routing table 48 from device E with the following entries (as shown in Fig. 7):
E via local with hop of 0;
D via direct with hop of 1; and
C via direct with hop of 1.

The above entries are updated by the agent 20 of device C to:
E via E with hop of 1;
D via E with hop of 2; and
C via E with hop of 2.

The original entries in the routing table 48 of device C from Fig. 7 are as follows:
1. C via local with hop of 0;
2. B via direct with hop of 1;
3. D via direct with hop of 1; and
4. E via direct with hop of 1.

The following entries are candidates for adding to the routing table 48 of device C based on the above:
5. D via D with hop of 1;
6. C via D with hop of 2;
7. E via D with hop of 2;
8. B via B with hop of 1;
9. Cloud via B with hop of 2;
10. A via B with hop of 2;
11. C via B with hop of 2;
12. E via E with hop of 1;
13. D via E with hop of 2; and
14. C via E with hop of 2.

The above 14 items may be analyzed for duplicates as follows.

Items 1, 6, 11, and 14 provide routing information to device C. Item 1 has the lowest hop value. Therefore, item 1 is retained while items 6, 11, and 14 are removed.

Items 2 and 8 provide routing information to device B. The entries are identical so either one may be removed.

Items 3, 5, and 13 provide routing information to device D. Items 3 and 5 have the lowest hop value and item 3 is via "direct". Therefore, item 3 is retained while items 5 and 13 are removed.

Items 4, 7, and 12 provide routing information to device E. Items 4 and 12 have the lowest hop value and item 4 is via "direct". Therefore, item 4 is retained while items 7 and 12 are removed.

Item 10 provides the routing information to device A and is therefore retained.

Item 9 provides the routing information to the cloud and is therefore retained.

Removing the abovementioned items leaves the following items in the routing table 48 of device C:
1. C via local with hop of 0;
2. B via direct with hop of 1;
3. D via direct with hop of 1;
4. E via direct with hop of 1.
9. Cloud via B with hop of 2; and
10. A via B with hop of 2.

The above also corresponds to the entries shown in the routing table 48 of device C in Fig. 8.

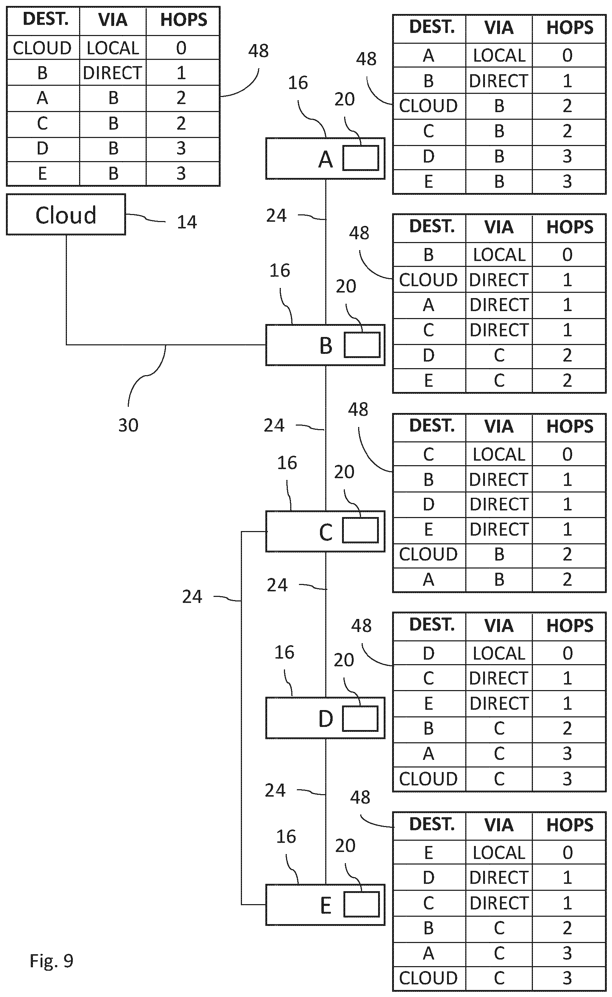

The updated routing tables 48 are again shared with direct neighbors and processed as above to update the "via" and "hop" values as appropriate and remove duplicates based on retaining the entries with the shortest hops yielding the routing tables 48 shown in Fig. 9.

In some implementations, the routing table 48 may need to be shared multiple times and processed as described above until a steady state is reached.

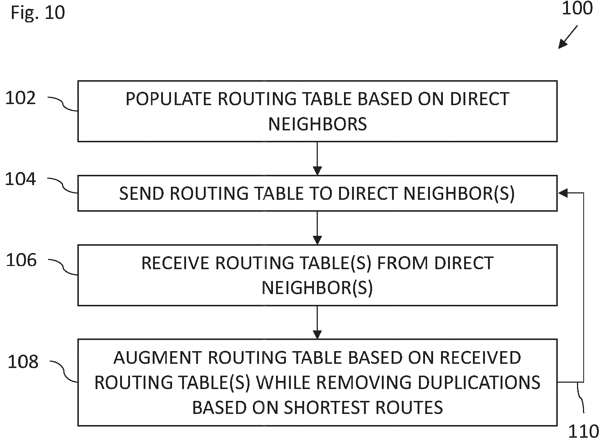

Reference is also made to Fig. 10, which is a flowchart 100 including steps in a method of generating routing tables 48 for use in the system 10 of Fig. 1. The agent 20 is configured to populate the routing table 48 (i.e., its local routing table 48) based on its own device and the list 74 of the one or more direct neighbors (block 102). The agent 20 is configured to send the routing table 48 (i.e., its local routing table 48) to the one or more direct neighbors (block 104) and receive routing table(s) 48 from the direct neighbor(s) (block 106). The agent 20 is configured to augment the routing table 48 (i.e., its local routing table 48) based on data included in the received routing table(s) (e.g., by updating the "via" value to the ID of the device sending the routing table 48 and by increasing the "hop" value by 1 for each entry in the received routing table(s) 48), while removing entries of one or more duplicate destination medical devices based on consideration of shortest routes to the duplicate destination medical devices (block 108) by retaining the entries with the shortest routes (i.e. lowest number of hops). The steps of blocks 104-108 may need to be repeated one or more times (arrow 110) until the routing table 48 of each medical device 16 and the cloud medical device software update agent(s) 14 are in steady state (i.e., do not change).

Reference is now made to Fig. 11, which is a flowchart 200 including steps in a method of providing software updates to one of the devices 16 of Fig. 4. The terms local agent 20, and local medical device software 22 are used to refer to the agent 20 and medical device software 22 running on the same local medical device 16.

The local agent 20 running in the local medical device 16 is configured to receive identity information 42 about the local medical device 16 from the local medical device software 22 (block 202). The local agent 20 is configured to send the identity information 42 to the cloud medical device software update agent 14 via the local interface 34 and one or more other agents 20 installed in one or more other corresponding medical devices 16 (block 204). The cloud medical device software update agent 14 is configured to receive the identity information 42, sign the software update 26 with the private key of the manufacturer of the local medical device 16, optionally encrypt the software update 26 with the public key of the local medical device 16, and send the software update 26 and its signature to the local medical device 16 via the other agent(s) 20 (block 206). The local agent 20 is configured to receive, from the cloud medical device software update agent 14 via the other agent(s) 20 installed in the other medical device(s) 16, the medical device software update 26 and the digital signature of the medical device software update 26 signed by the manufacturer of local medical device 16 (block 208).

The processor 36 or the agent 20 of the local medical device 16 is configured to authenticate the received medical device software update 26 based on the digital signature (e.g., public key of the manufacturer) (block 210). When the medical device software update 26 is encrypted by the manufacturer of the local medical device 16 using a public key of the local medical device 16, the processor 36 of the medical device 16 is configured to decrypt the medical device software update 26 using the private key of the local medical device 16 (e.g., stored in the tamper resistant chip 40 (Fig. 4)) (block 210).

At a decision block 212, the processor 36 or the agent 20 of the local medical device 16 is configured to confirm if the software update 26 is authenticated. If the software updates 26 is authenticated, the processor 36 of the local medical device 16 is configured to install the authenticated medical device software update 26 (block 214).

Once the local agent 20 sends the software update 26 to the medical device software 22, the medical device software 22 may notify the user of the local medical device 16, so the user will select an appropriate time to install the update. Alternatively, the update may be installed automatically when the device is idle. In this case, the local medical device 16 may offer an option to cancel the update. If there is an emergency and the hospital staff needs the device immediately, they can cancel the update during the installation and use the medical device. Alternatively, the update may be installed automatically during non-working hours. In this case, too, the local medical device 16 may offer an option to cancel the update.

If the processor 36 of the local medical device 16 checks the digital signature and determines that the digital signature does not authenticate the medical device software update 26, and another medical device 16 was meant to have first checked the digital signature, then the local agent 20 may be configured to block files provided by the other medical device 16 based on a failure of the other medical device to identify lack of authentication of the medical device software update 26.

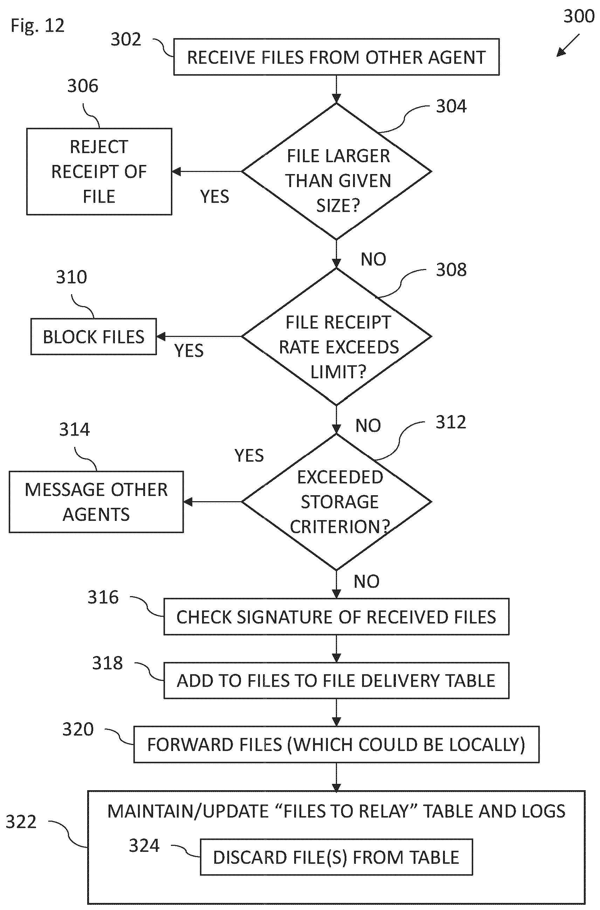

Reference is now made to Fig. 12, which is a flowchart 300 including steps in a method of receiving and processing files in the local medical device 16 of Fig. 4.

The local agent 20 of the local medical device 16 is configured to receive files including one or more medical device software updates and one or more corresponding digital signatures, via one or more other agents 20, destined for one or more other medical devices or for the local medical device 16 (block 302).

In some exemplary modes, at a decision block 304, the local agent 20 is configured to check if the file is larger than a given size. The local agent 20 is configured to reject receipt of a file larger than a given size limit from other medical device software update agents 20 (block 306).

In some exemplary modes, at a decision block 308, the local agent 20 is configured to check if the file receipt rate exceeds a limit. The local agent 20 is configured to block files (e.g., for a period of time) provided by a given medical device 16 based on the given medical device providing more than a given number of files in a given time period (block 310).

In some exemplary modes, at a decision block 312, the local agent 20 is configured to check if the local medical device 16 has exceeded a storage criterion (e.g., disk full or exceeded limit or queue full). The local agent 20 is configured to send messages to the other medical device software update agents 20 to remove the local medical device 16 from active routing data (e.g., remove from the routing tables 48) in response to exceeding the storage criterion (block 314).

In some exemplary modes, the local agent 20 is configured to authenticate the received medical device software updates based on the received digital signatures (block 316) and delete files which fail the authentication process. The local agent 20 is configured to add the details of the received files to the file delivery table 46 (block 318). The local agent 20 is configured to forward some, or all, of the files received via the local agent 20 from the cloud medical device software update agent 14 to other medical device(s) 16 in the network based on the corresponding destination information 56 (Fig. 5) of the files and the corresponding routing information 66 (Fig. 5) of the assessable medical devices in the routing table 48 stored in the memory 38 (block 320). One or more of the files received may be forwarded to the local medical device software 22 for installation. In exemplary modes where the step of block 316 is performed, the step of block 320 is performed based on the files (e.g., medical device software updates) being authenticated.

The local agent 20 is configured to maintain a queue of stored files for forwarding to medical device(s) 16, for example, using the file delivery table 46 (block 322), and discard files from the medical device 16 according to a purging policy of the queue, for example, purging files older than a given value, or purging files on a FIFO basis when the queue size or total file size reaches a given value (block 324). The step of block 322 may include the local agent 20 generating a log to track received files, files forwarded to other medical devices 16, files forwarded to the local medical device software 22 for installation etc.

Reference is now made to Fig. 13, which is a flowchart 400 including steps in a method of receiving and processing logs in the local medical device 16 of Fig. 4. The local agent 20 is configured to receive a log of software installation and/or software update from the local medical device software 22 running on the local medical device 16 (block 402). The local agent 20 is configured to digitally sign the received log using a private key of the local medical device 16 for checking by the manufacturer of the local medical device 16 using a public key of the local medical device 16 (block 404). The local agent 20 is configured to optionally encrypt the log with a public key of the manufacturer of the medical device 16. The local agent 20 is configured to receive logs from one or more other medical devices for forwarding to the cloud medical device software update agent 14 (block 406). The local agent 20 is configured to forward the received logs to the cloud medical device software update agent 14 via the interface 34 and one or more agents 20 installed in one or more corresponding other medical devices 16 (block 408).

Reference is now made to Fig. 14, which is a flowchart 500 including steps in a method of receiving and processing logs received by one of the cloud medical device software update agents 14 in the system 10 of Fig. 1. The cloud medical device software update agent 14 is configured to: receive logs from the agents 20 of the medical devices 16 (block 502); authenticate (e.g., with corresponding public keys of the sending medical devices 16) and optionally decrypt the logs (e.g., with corresponding private keys of the manufacturers of the sending medical devices 16) (block 504); identify a failure of a given software update agent 20 to relay files from the cloud medical device software update agent 14 based on the received logs (block 506); and perform an action (such as removing the device of that agent from the routing tables 48 for a given time period) with respect to the given software update agent 20 responsively to identifying the failure (block 508).

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%.

Various features of the disclosure which are, for clarity, described in the contexts of separate exemplary modes may also be provided in combination in a single exemplary mode. Conversely, various features of the disclosure which are, for brevity, described in the context of a single exemplary mode may also be provided separately or in any suitable sub-combination.

The exemplary modes described above are cited by way of example, and the present disclosure is not limited by what has been particularly shown and described hereinabove. Rather the scope of the disclosure includes both combinations and sub-combinations of the various features described hereinabove, as well as variations and modifications thereof which would occur to persons skilled in the art upon reading the foregoing description and which are not disclosed in the prior art, provided they fall within the scope of the claims.

## Claims

1. A medical system (10), comprising:
a first medical device (16), including an interface (34) configured to connect the first medical device (16) to a network of multiple medical devices (16);
the multiple medical devices (16), wherein the first medical device (16) and the multiple medical devices (16) are installed in a medical facility; and
a cloud medical device software update agent;
wherein a given one of the multiple medical devices (16) is configured to establish an internet connection (30) with the cloud medical device software update agent (14);
wherein the first medical device further comprises a processor (36) configured to:
run medical device software (22);
run a first medical device software update agent (20) configured to:
receive identity information (42) about the first medical device (16) from the medical device software;
send the identity information (42) to the cloud medical device software update agent (14) via the interface (34) and at least one second medical device software update agent (20) installed in at least one second medical device (16) of the multiple medical devices (16); and
receive, from the cloud medical device software update agent (14) via the at least one second medical device software update agent (20) installed in the at least one second medical device (16), a medical device software update (26) and a digital signature of the medical device software update (26) signed by a manufacturer of the first medical device (16);
authenticate the received medical device software update (26) based on the digital signature; and
install the authenticated medical device software update (26); and wherein the cloud medical device software update agent (14) is configured to:
receive logs (28) from the first medical device software update agent (20) and software update agents (20) of the multiple medical devices (16);
identify failure of a given one of the software update agents (20) to relay files from the cloud medical device software update agent (14) based on the received logs (28); and
perform an action with respect to the given software update agent (20) responsively to identifying the failure.

2. The system according to claim 1, further comprising the cloud medical device software update agent (14) of a first medical device manufacturer and another cloud medical device software update agent (14) of a second medical device manufacturer, which is configured to connect to the cloud medical device software update agent (14) of the first medical device manufacturer via another internet connection (30).

3. The system according to claim 2, wherein the cloud medical device software update agent (14) of the first medical device manufacturer and the other cloud medical device software update agent (14) of the second medical device manufacturer are configured to be connected to a medical device (16-1) of the first medical device manufacturer in the network of multiple medical devices (16) via a first internet connection (30), and to a medical device (16-2) of the second medical device manufacturer in the network of multiple medical devices (16) via a second internet connection (30), respectively.

4. The system according to any of claims 1-3, wherein the first medical device (16) includes a memory (38) configured to store:
a file delivery table (46) including:
indications (54) of one or more files received from the cloud medical device software update agent (14) for delivery to one or more of the multiple medical devices (16) in the network; and
corresponding destination information (56) of the one or more files; and
a routing table (48) including:
identification information (64) of assessable ones of the medical devices (16) in the network; and
corresponding routing information (66) of the assessable medical devices.

5. The system according to claim 4, wherein the first medical device software update agent (20) is configured to forward the one or more files received via the at least one second medical device software update agent (20) from the cloud medical device software update agent (14) to the one or more multiple medical devices (16) in the network based on the corresponding destination information (56) of the one or more files and the corresponding routing information (66) of the assessable medical devices in the routing table (48).

6. The system according to claim 4 or 5, wherein the memory (38) configured to store a direct neighbor table (52) including:
a list of one or more direct neighbors (74) of the first medical device (16), and
address information (76) identifying the one or more direct neighbors in the network of medical devices (16).

7. The system according to claim 6, wherein the first medical device software update agent (20) is configured to:
populate the routing table (48) based on the list of the one or more direct neighbors (74);
send the routing table (48) to the one or more direct neighbors; and
receive one or more routing tables (48) from the one or more direct neighbors; and
augment the routing table (48) based on data included in the received one or more routing tables (48) while removing one or more duplicate destination medical devices (16) based on consideration of shortest routes to the one or more duplicate destination medical devices (16).

8. The system according to any of claims 1-7, wherein the first medical device software update agent (20) is configured to receive, an additional medical device software update (26) and an additional corresponding digital signature from a given medical device (16) of the multiple medical devices (16), wherein:
the processor (36) is configured to check the additional digital signature and determine that the additional digital signature does not authenticate the additional medical device software update (26); and
the first medical device software update agent (20) is configured to block files provided by the given medical device (16) based on a failure of the given medical device (16) to identify lack of authentication of the additional medical device software update (26).

9. The system according to any of claims 1-8, wherein the first medical device software update agent (20) is configured to:
receive an additional medical device software update (26) and an additional digital signature, via the at least one second medical device software update agent (20) from the cloud medical device software update agent (14), destined for a given medical device (16) of the multiple medical devices (16);
authenticate the received additional medical device software update (26) based on the additional digital signature; and
forward the additional medical device software update (26) to the given medical device (16) responsively to authenticating the additional medical device software update (26) based on the additional digital signature.

10. The system according to any of claims 1-9, wherein the first medical device software update agent (20) is configured to send messages to the other medical device software update agents (20) to remove the first medical device (16) from active routing data in response to exceeding a storage criterion.

11. The system according to any of claims 1-10, wherein the first medical device software update agent (20) is configured to:
receive a log (28) of software installation and/or software update from the medical device software (22) running on the first medical device (16); and
forward the received log (28) to the cloud medical device software update agent (14) via the interface (34) and the at least one second medical device software update agent (20).

12. The system according to claim 1, wherein the first medical device software update agent (20) is configured to:
receive logs (28) from at least one of the multiple medical devices (16); and
forward the received logs (28) to the cloud medical device software update agent (14) via the at least one second medical device software update agent (20).

13. A medical method, comprising:
connecting a first medical device (16) to a network of multiple medical devices (16), wherein the first medical device (16) and the multiple medical devices (16) are installed in a medical facility and a given one of the multiple medical devices (16) establishes an internet connection (30) with a cloud medical device software update agent (14);
running medical device software (22) by the first medical device;
running a first medical device software update agent (20) by the first medical device;
receiving (202), by first medical device software update agent (20), identity information (42) about the first medical device (16) from the medical device software (22);
sending (204), by first medical device software update agent (20), the identity information (42) to the cloud medical device software update agent (14) via at least one second medical device software update agent (20) installed in at least one second medical device (16) of the multiple medical devices (16);
receiving (208), by first medical device software update agent (20), from the cloud medical device software update agent (14) via the at least one second medical device software update agent (20) installed in the at least one second medical device (16), a medical device software update (26) and a digital signature of the medical device software update (26) signed by a manufacturer of the first medical device (16);
authenticating (210) the received medical device software update (26) based on the digital signature;
installing (214) the authenticated medical device software update (26);
receiving logs, by the cloud medical device software update agent (14), from the first medical device software agent (20) and software update agents (20) of the multiple medical devices (16);
identifying, by the cloud medical device software update agent (14), failure of a given one of the software update agents (20) to relay files from the cloud medical device software update agent (14) based on the received logs (28); and
performing, by the cloud medical device software update agent (14), an action with respect to the given software update agent (20) responsively to identifying the failure.

## Patentansprüche

1. Medizinisches System (10), umfassend:
eine erste medizinische Vorrichtung (16), einschließlich einer Schnittstelle (34), die konfiguriert ist, um die erste medizinische Vorrichtung (16) mit einem Netzwerk aus mehreren medizinischen Vorrichtungen (16) zu verbinden;
die mehreren medizinischen Vorrichtungen (16), wobei die erste medizinische Vorrichtung (16) und die mehreren medizinischen Vorrichtungen (16) in einer medizinischen Einrichtung installiert sind; und
einen Cloud-Softwareupdate-Agenten für medizinische Vorrichtungen;
wobei eine gegebene der mehreren medizinischen Vorrichtungen (16) konfiguriert ist, um mit dem Cloud-Softwareupdate-Agenten für medizinische Vorrichtungen (14) eine Internetverbindung (30) herzustellen;
wobei die erste medizinische Vorrichtung ferner einen Prozessor (36) umfasst, der konfiguriert ist, um:
Software (22) für eine medizinische Vorrichtung auszuführen;
einen ersten Softwareupdate-Agenten für medizinische Vorrichtungen (20) auszuführen, der konfiguriert ist, um:
Identitätsinformationen (42) über die erste medizinische Vorrichtung (16) von der Software medizinischer Vorrichtungen zu empfangen;
die Identitätsinformationen (42) an den Cloud-Softwareupdate-Agenten für medizinische Vorrichtungen (14) über die Schnittstelle (34) und mindestens einen zweiten Softwareupdate-Agenten für medizinische Vorrichtungen (20), der in mindestens einer zweiten medizinischen Vorrichtung (16) der mehreren medizinischen Vorrichtungen (16) installiert ist, zu senden; und
von dem Cloud-Softwareupdate-Agenten für medizinische Vorrichtungen (14) über den mindestens einen zweiten Softwareupdate-Agenten für medizinische Vorrichtungen (20), der in der mindestens einen zweiten medizinischen Vorrichtung (16) installiert ist, ein Softwareupdate für medizinische Vorrichtungen (26) und eine digitale Signatur des Softwareupdates für medizinische Vorrichtungen (26), die von einem Hersteller der ersten medizinischen Vorrichtung (16) signiert ist, zu empfangen;
das empfangene Softwareupdate für medizinische Vorrichtungen (26) basierend auf der digitalen Signatur zu authentifizieren; und
das authentifizierte Softwareupdate für medizinische Vorrichtungen (26) zu installieren; und
wobei der Cloud-Softwareupdate-Agent für medizinische Vorrichtungen (14) konfiguriert ist, um:
Protokolle (28) von dem ersten Softwareupdate-Agenten für medizinische Vorrichtungen (20) und Softwareupdate-Agenten (20) der mehreren medizinischen Vorrichtungen (16) zu empfangen;
Fehler eines bestimmten Softwareupdate-Agenten (20) beim Weiterleiten von Dateien von dem Cloud-Softwareupdate-Agenten für medizinische Vorrichtungen (14) basierend auf den empfangenen Protokollen (28) zu identifizieren; und
eine Aktion in Bezug auf den gegebenen Softwareupdate-Agenten (20) als Reaktion auf das Identifizieren des Fehlers durchzuführen.

2. System nach Anspruch 1, ferner umfassend den Cloud-Softwareupdate-Agenten für medizinische Vorrichtungen (14) eines ersten Herstellers medizinischer Vorrichtungen und einen weiteren Cloud-Softwareupdate-Agenten für medizinische Vorrichtungen (14) eines zweiten Herstellers medizinischer Vorrichtungen, der konfiguriert ist, um über eine weitere Internetverbindung (30) mit dem Cloud-Softwareupdate-Agenten für medizinische Vorrichtungen (14) des ersten Herstellers medizinischer Vorrichtungen zu verbinden.

3. System nach Anspruch 2, wobei der Cloud-Softwareupdate-Agent für medizinische Vorrichtungen (14) des ersten Herstellers medizinischer Vorrichtungen und der andere Cloud-Softwareupdate-Agent für medizinische Vorrichtungen (14) des zweiten Herstellers medizinischer Vorrichtungen konfiguriert sind, um über eine erste Internetverbindung (30) mit einer medizinischen Vorrichtung (16-1) des ersten Herstellers medizinischer Vorrichtungen in dem Netzwerk mehrerer medizinischer Vorrichtungen (16) bzw. über eine zweite Internetverbindung (30) mit einer medizinischen Vorrichtung (16-2) des zweiten Herstellers medizinischer Vorrichtungen in dem Netzwerk mehrerer medizinischer Vorrichtungen (16) verbunden zu werden.

4. System nach einem der Ansprüche 1 bis 3, wobei die erste medizinische Vorrichtung (16) einen Speicher (38) einschließt, der konfiguriert ist, um zu speichern:
eine Dateizustellungstabelle (46), einschließlich:
Angaben (54) zu einer oder mehreren Dateien, die von dem Cloud-Softwareupdate-Agenten für medizinische Vorrichtungen (14) zur Zustellung an eine oder mehrere der mehreren medizinischen Vorrichtungen (16) in dem Netzwerk empfangen wurden; und
entsprechender Zielinformationen (56) der einen oder mehreren Dateien; und
eine Routing-Tabelle (48), einschließlich:
Identifikationsinformationen (64) der beurteilbaren medizinischen Vorrichtungen (16) in dem Netzwerk; und
entsprechender Routing-Informationen (66) der beurteilbaren medizinischen Vorrichtungen.

5. System nach Anspruch 4, wobei der erste Softwareupdate-Agent für medizinische Vorrichtungen (20) konfiguriert ist, um die eine oder mehreren Dateien, die über den mindestens einen zweiten Softwareupdate-Agenten für medizinische Vorrichtungen (20) von dem Cloud-Softwareupdate-Agenten für medizinische Vorrichtungen (14) empfangen werden, an die eine oder mehreren Vorrichtungen (16) basierend auf entsprechenden Zielinformationen (56) der einen oder mehreren Dateien und der entsprechenden Routing-Information (66) der beurteilbaren medizinischen Vorrichtungen in der Routing-Tabelle (48) in dem Netzwerk weiterzuleiten.

6. System nach Anspruch 4 oder 5, wobei der Speicher (38) konfiguriert ist, um eine direkte Nachbartabelle (52) zu speichern, einschließlich:
einer Liste eines oder mehrerer direkter Nachbarn (74) der ersten medizinischen Vorrichtung (16), und
Adressinformationen (76), die den einen oder die mehreren direkten Nachbarn in dem Netzwerk medizinischer Vorrichtungen (16) identifizieren.

7. System nach Anspruch 6, wobei der erste Softwareupdate-Agent für medizinische Vorrichtungen (20) konfiguriert ist, um:
die Routing-Tabelle (48) basierend auf der Liste des einen oder der mehreren direkten Nachbarn (74) zu füllen;
die Routing-Tabelle (48) an den einen oder die mehreren direkten Nachbarn zu senden; und
eine oder mehrere Routing-Tabellen (48) von dem einen oder den mehreren direkten Nachbarn zu empfangen; und
die Routing-Tabelle (48) basierend auf Daten, die in der empfangenen einen oder mehreren Routing-Tabellen (48) eingeschlossen sind, zu ergänzen, während eine oder mehrere doppelte medizinische Zielvorrichtungen (16) basierend auf der Berücksichtigung der kürzesten Routen zu der einen oder den mehreren doppelten medizinischen Zielvorrichtungen (16), entfernt werden.

8. System nach einem der Ansprüche 1 bis 7, wobei der erste Softwareupdate-Agent für medizinische Vorrichtungen (20) konfiguriert ist, um ein zusätzliches Softwareupdate für medizinische Vorrichtungen (26) und eine zusätzliche entsprechende digitale Signatur von einer gegebenen medizinischen Vorrichtung (16) der mehreren medizinischen Vorrichtungen (16) zu empfangen, wobei:
der Prozessor (36) konfiguriert ist, um die zusätzliche digitale Signatur zu prüfen und zu ermitteln, dass die zusätzliche digitale Signatur das zusätzliche Softwareupdate der medizinischen Vorrichtung (26) nicht authentifiziert; und
der erste Softwareupdate-Agent für medizinische Vorrichtungen (20) konfiguriert ist, um die von der gegebenen medizinischen Vorrichtung (16) bereitgestellten Dateien basierend auf einem Fehler der gegebenen medizinischen Vorrichtung (16), fehlende Authentifizierung des zusätzlichen Softwareupdates für medizinische Vorrichtungen (26) zu identifizieren, zu blockieren.

9. System nach einem der Ansprüche 1 bis 8, wobei der erste Softwareupdate-Agent für medizinische Vorrichtungen (20) konfiguriert ist, um:
ein zusätzliches Softwareupdate für medizinische Vorrichtungen (26) und eine zusätzliche digitale Signatur über den mindestens einen zweiten Softwareupdate-Agenten für medizinische Vorrichtungen (20) zu empfangen, von dem Cloud-Softwareupdate-Agenten für medizinische Vorrichtungen (14), die für eine gegebene medizinische Vorrichtung (16) der mehreren medizinischen Vorrichtungen (16) bestimmt ist;
das empfangene zusätzliche Softwareupdate für medizinische Vorrichtungen (26) basierend auf der zusätzlichen digitalen Signatur zu authentifizieren; und
das zusätzliche Softwareupdate für medizinische Vorrichtungen (26) an die gegebene medizinische Vorrichtung (16) als Reaktion auf das Authentifizieren des zusätzlichen Softwareupdate für medizinische Vorrichtungen (26) basierend auf der zusätzlichen digitalen Signatur weiterzuleiten.

10. System nach einem der Ansprüche 1 bis 9, wobei das erste Softwareupdate-Agent für medizinische Vorrichtungen (20) konfiguriert ist, um Nachrichten an die anderen Softwareupdate-Agenten für medizinische Vorrichtungen (20) zu senden, um die erste medizinische Vorrichtung (16) als Reaktion auf das Überschreiten eines Speicherkriteriums aus den aktiven Routing-Daten zu entfernen.

11. System nach einem der Ansprüche 1 bis 10, wobei der erste Softwareupdate-Agent für medizinische Vorrichtungen (20) konfiguriert ist, um:
ein Protokoll (28) der Softwareinstallation und/oder des Softwareupdates von der Software für medizinische Vorrichtungen (22), die auf der ersten medizinischen Vorrichtung (16) läuft, zu empfangen; und
das empfangene Protokoll (28) an den Cloud-Softwareupdate-Agenten für medizinische Vorrichtungen (14) über die Schnittstelle (34) und den mindestens einen zweiten Softwareupdate-Agenten für medizinische Vorrichtungen (20) weiterzuleiten.

12. System nach Anspruch 1, wobei der erste Softwareupdate-Agent für medizinische Vorrichtungen (20) konfiguriert ist, um:
Protokolle (28) von mindestens einer der mehreren medizinischen Vorrichtungen (16) zu empfangen; und
die empfangenen Protokolle (28) an den Cloud-Softwareupdate-Agenten für medizinische Vorrichtungen (14) über den mindestens einen zweiten Softwareupdate-Agenten für medizinische Vorrichtungen (20) weiterzuleiten.

13. Medizinisches Verfahren, umfassend:
Verbinden einer ersten medizinischen Vorrichtung (16) mit einem Netzwerk mehrerer medizinischer Vorrichtungen (16), wobei die erste medizinische Vorrichtung (16) und die mehreren medizinischen Vorrichtungen (16) in einer medizinischen Einrichtung installiert sind und eine gegebene der mehreren medizinischen Vorrichtungen (16) eine Internetverbindung (30) mit einem Cloud-Softwareupdate-Agenten für medizinische Vorrichtungen (14) herstellt;
Ausführen von Software für medizinische Vorrichtungen (22) durch die erste medizinische Vorrichtung;
Ausführen eines ersten Softwareupdate-Agenten für medizinische Vorrichtungen (20) durch die erste medizinische Vorrichtung;
Empfangen (202) von der Software für medizinische Vorrichtungen (22), durch den ersten Softwareupdate-Agenten für medizinische Vorrichtungen (20), von Identitätsinformationen (42) über die erste medizinische Vorrichtung (16);
Senden (204), durch den ersten Softwareupdate-Agenten für medizinische Vorrichtungen (20), der Identitätsinformationen (42) an den Cloud-Softwareupdate-Agenten für medizinische Vorrichtungen (14) über mindestens einen zweiten Softwareupdate-Agenten für medizinische Vorrichtungen (20), der in mindestens einer zweiten medizinischen Vorrichtung (16) der mehreren medizinischen Vorrichtungen (16) installiert ist;
Empfangen (208), durch den ersten Softwareupdate-Agenten für medizinische Vorrichtungen (20), von dem Cloud-Softwareupdate-Agenten für medizinische Vorrichtungen (14) über den mindestens einen zweiten Softwareupdate-Agenten für medizinische Vorrichtungen (20), der in der mindestens einen zweiten medizinischen Vorrichtung (16) installiert ist, eines Softwareupdates für medizinische Vorrichtungen (26) und einer digitalen Signatur des Softwareupdates für medizinische Vorrichtungen (26), die von einem Hersteller der ersten medizinischen Vorrichtung (16) signiert ist;
Authentifizieren (210) des empfangenen Softwareupdates für medizinische Vorrichtungen (26) basierend auf der digitalen Signatur;
Installieren (214) des authentifizierten Softwareupdates für medizinische Vorrichtungen (26);
Empfangen von Protokollen, durch den Cloud-Softwareupdate-Agenten für medizinische Vorrichtungen (14), von dem ersten Softwareupdate-Agenten für medizinische Vorrichtungen (20) und Softwareupdate-Agenten (20) der mehreren medizinischen Vorrichtungen (16);
Identifizieren, durch den Cloud-Softwareupdate-Agenten für medizinische Vorrichtungen (14), eines Fehlers eines gegebenen einen der Softwareupdate-Agenten (20) beim Weiterleiten von Dateien von dem Cloud-Softwareupdate-Agenten für medizinische Vorrichtungen (14) basierend auf den empfangenen Protokollen (28); und
Ausführen, durch den Cloud-Softwareupdate-Agenten für medizinische Vorrichtungen (14), einer Aktion in Bezug auf den gegebenen Softwareupdate-Agenten (20) als Reaktion auf das Identifizieren des Fehlers.

## Revendications

1. Système médical (10), comprenant :
un premier dispositif médical (16), comportant une interface (34) configurée pour connecter le premier dispositif médical (16) à un réseau de multiples dispositifs médicaux (16) ;
les multiples dispositifs médicaux (16), dans lequel le premier dispositif médical (16) et les multiples dispositifs médicaux (16) sont installés dans un établissement médical ; et
un agent de mise à jour de logiciel de dispositif médical en nuage ;
dans lequel un dispositif donné des multiples dispositifs médicaux (16) est configuré pour établir une connexion Internet (30) avec l'agent de mise à jour de logiciel de dispositif médical en nuage (14) ;
dans lequel le premier dispositif médical comprend en outre un processeur (36) configuré pour :
exécuter un logiciel de dispositif médical (22) ;
exécuter un premier agent de mise à jour de logiciel de dispositif médical (20) configuré pour :
recevoir des informations d'identité (42) concernant le premier dispositif médical (16) en provenance du logiciel de dispositif médical ;
envoyer les informations d'identité (42) à l'agent de mise à jour de logiciel de dispositif médical en nuage (14) par l'intermédiaire de l'interface (34) et d'au moins un second agent de mise à jour de logiciel de dispositif médical (20) installé dans au moins un second dispositif médical (16) des multiples dispositifs médicaux (16) ; et
recevoir, en provenance de l'agent de mise à jour de logiciel de dispositif médical en nuage (14) par l'intermédiaire de l'au moins un second agent de mise à jour de logiciel de dispositif médical (20) installé dans l'au moins un second dispositif médical (16), une mise à jour de logiciel de dispositif médical (26) et une signature numérique de la mise à jour de logiciel de dispositif médical (26) signée par un fabricant du premier dispositif médical (16) ;
authentifier la mise à jour de logiciel de dispositif médical (26) reçue en fonction de la signature numérique ; et
installer la mise à jour de logiciel de dispositif médical (26) authentifiée ; et
dans lequel l'agent de mise à jour de logiciel de dispositif médical en nuage (14) est configuré pour :
recevoir des journaux (28) en provenance du premier agent de mise à jour de logiciel de dispositif médical (20) et d'agents de mise à jour de logiciel (20) des multiples dispositifs médicaux (16) ;
identifier une incapacité d'un agent donné parmi les agents de mise à jour de logiciel (20) à relayer des fichiers à partir de l'agent de mise à jour de logiciel de dispositif médical en nuage (14) en fonction des journaux (28) reçus ; et
mettre en oeuvre une action par rapport à l'agent de mise à jour de logiciel (20) donné en réponse à l'identification de l'incapacité.

2. Système selon la revendication 1, comprenant en outre l'agent de mise à jour de logiciel de dispositif médical en nuage (14) d'un premier fabricant de dispositif médical et un autre agent de mise à jour de logiciel de dispositif médical en nuage (14) d'un second fabricant de dispositif médical, qui est configuré pour se connecter à l'agent de mise à jour de logiciel de dispositif médical en nuage (14) du premier fabricant de dispositif médical par l'intermédiaire d'une autre connexion Internet (30).

3. Système selon la revendication 2, dans lequel l'agent de mise à jour de logiciel de dispositif médical en nuage (14) du premier fabricant de dispositif médical et l'autre agent de mise à jour de logiciel de dispositif médical en nuage (14) du second fabricant de dispositif médical sont configurés pour être connectés à un dispositif médical (16-1) du premier fabricant de dispositif médical dans le réseau de multiples dispositifs médicaux (16) par l'intermédiaire d'une première connexion Internet (30), et à un dispositif médical (16-2) du second fabricant de dispositif médical dans le réseau de multiples dispositifs médicaux (16) par l'intermédiaire d'une seconde connexion Internet (30), respectivement.

4. Système selon l'une quelconque des revendications 1 à 3, dans lequel le premier dispositif médical (16) comporte une mémoire (38) configurée pour stocker :
une table de distribution de fichiers (46) comportant :
des indications (54) d'un ou plusieurs fichiers reçus en provenance de l'agent de mise à jour de logiciel de dispositif médical en nuage (14) pour distribution à un ou plusieurs des multiples dispositifs médicaux (16) dans le réseau ; et
des informations de destination correspondantes (56) du ou des fichiers ; et
une table de routage (48) comportant :
des informations d'identification (64) de dispositifs médicaux évaluables parmi les dispositifs médicaux (16) dans le réseau ; et
des informations de routage correspondantes (66) des dispositifs médicaux évaluables.

5. Système selon la revendication 4, dans lequel le premier agent de mise à jour de logiciel de dispositif médical (20) est configuré pour acheminer le ou les fichiers reçus par l'intermédiaire de l'au moins un second agent de mise à jour de logiciel de dispositif médical (20) de l'agent de mise à jour de logiciel de dispositif médical en nuage (14) vers le ou les multiples dispositifs médicaux (16) dans le réseau en fonction des informations de destination correspondantes (56) du ou des fichiers et des informations de routage correspondantes (66) des dispositifs médicaux évaluables dans la table de routage (48).

6. Système selon la revendication 4 ou 5, dans lequel la mémoire (38) est configurée pour stocker une table de voisins directs (52) comportant :
une liste d'un ou plusieurs voisins directs (74) du premier dispositif médical (16), et
des informations d'adresse (76) identifiant le ou les voisins directs dans le réseau de dispositifs médicaux (16).

7. Système selon la revendication 6, dans lequel le premier agent de mise à jour de logiciel de dispositif médical (20) est configuré pour :
peupler la table de routage (48) en fonction de la liste du ou des voisins directs (74) ;
envoyer la table de routage (48) au ou aux voisins directs ; et
recevoir une ou plusieurs tables de routage (48) en provenance du ou des voisins directs ; et
enrichir la table de routage (48) en fonction de données incluses dans la ou les tables de routage (48) reçues tout en retirant un ou plusieurs dispositifs médicaux (16) de destination en double en fonction d'une considération de routes les plus courtes vers le ou les dispositifs médicaux (16) de destination en double.

8. Système selon l'une quelconque des revendications 1 à 7, dans lequel le premier agent de mise à jour de logiciel de dispositif médical (20) est configuré pour recevoir, une mise à jour de logiciel de dispositif médical (26) supplémentaire et une signature numérique correspondante supplémentaire en provenance d'un dispositif médical (16) donné parmi les multiples dispositifs médicaux (16), dans lequel :
le processeur (36) est configuré pour vérifier la signature numérique supplémentaire et déterminer que la signature numérique supplémentaire n'authentifie pas la mise à jour de logiciel de dispositif médical (26) supplémentaire ; et
le premier agent de mise à jour de logiciel de dispositif médical (20) est configuré pour bloquer des fichiers fournis par le dispositif médical (16) donné en fonction d'une incapacité du dispositif médical (16) donné à identifier une absence d'authentification de la mise à jour de logiciel de dispositif médical (26) supplémentaire.

9. Système selon l'une quelconque des revendications 1 à 8, dans lequel le premier agent de mise à jour de logiciel de dispositif médical (20) est configuré pour :
recevoir une mise à jour de logiciel de dispositif médical (26) supplémentaire et une signature numérique supplémentaire, par l'intermédiaire de l'au moins un second agent de mise à jour de logiciel de dispositif médical (20) en provenance de l'agent de mise à jour de logiciel de dispositif médical en nuage (14), destinées à un dispositif médical (16) donné parmi les multiples dispositifs médicaux (16) ;
authentifier la mise à jour de logiciel de dispositif médical (26) supplémentaire reçue en fonction de la signature numérique supplémentaire ; et
acheminer la mise à jour de logiciel de dispositif médical (26) supplémentaire vers le dispositif médical (16) donné en réponse à l'authentification de la mise à jour de logiciel de dispositif médical (26) supplémentaire en fonction de la signature numérique supplémentaire.

10. Système selon l'une quelconque des revendications 1 à 9, dans lequel le premier agent de mise à jour de logiciel de dispositif médical (20) est configuré pour envoyer des messages aux autres agents de mise à jour de logiciel de dispositif médical (20) pour retirer le premier dispositif médical (16) de données de routage actif en réponse à un dépassement d'un critère de stockage.

11. Système selon l'une quelconque des revendications 1 à 10, dans lequel le premier agent de mise à jour de logiciel de dispositif médical (20) est configuré pour :
recevoir un journal (28) d'installation de logiciel et/ou de mise à jour de logiciel en provenance du logiciel de dispositif médical (22) en cours d'exécution sur le premier dispositif médical (16) ; et
acheminer le journal (28) reçu vers l'agent de mise à jour de logiciel de dispositif médical en nuage (14) par l'intermédiaire de l'interface (34) et de l'au moins un second agent de mise à jour de logiciel de dispositif médical (20).

12. Système selon la revendication 1, dans lequel le premier agent de mise à jour de logiciel de dispositif médical (20) est configuré pour :
recevoir des journaux (28) en provenance d'au moins l'un des multiples dispositifs médicaux (16) ; et
acheminer les journaux (28) reçus vers l'agent de mise à jour de logiciel de dispositif médical en nuage (14) par l'intermédiaire de l'au moins un second agent de mise à jour de logiciel de dispositif médical (20).

13. Procédé médical, comprenant :
la connexion d'un premier dispositif médical (16) à un réseau de multiples dispositifs médicaux (16), dans lequel le premier dispositif médical (16) et les multiples dispositifs médicaux (16) sont installés dans un établissement médical et un dispositif médical donné des multiples dispositifs médicaux (16) établit une connexion Internet (30) avec un agent de mise à jour de logiciel de dispositif médical en nuage (14) ;
l'exécution d'un logiciel de dispositif médical (22) par le premier dispositif médical ;
l'exécution d'un premier agent de mise à jour de logiciel de dispositif médical (20) par le premier dispositif médical ;
la réception (202), par le premier agent de mise à jour de logiciel de dispositif médical (20), d'informations d'identité (42) concernant le premier dispositif médical (16) en provenance du logiciel de dispositif médical (22) ;
l'envoi (204), par le premier agent de mise à jour de logiciel de dispositif médical (20), des informations d'identité (42) à l'agent de mise à jour de logiciel de dispositif médical en nuage (14) par l'intermédiaire d'au moins un second agent de mise à jour de logiciel de dispositif médical (20) installé dans au moins un second dispositif médical (16) des multiples dispositifs médicaux (16) ;
la réception (208), par le premier agent de mise à jour de logiciel de dispositif médical (20), en provenance de l'agent de mise à jour de logiciel de dispositif médical en nuage (14) par l'intermédiaire de l'au moins un second agent de mise à jour de logiciel de dispositif médical (20) installé dans l'au moins un second dispositif médical (16), d'une mise à jour de logiciel de dispositif médical (26) et d'une signature numérique de la mise à jour de logiciel de dispositif médical (26) signée par un fabricant du premier dispositif médical (16) ;
l'authentification (210) de la mise à jour de logiciel de dispositif médical (26) reçue en fonction de la signature numérique ;
l'installation (214) de la mise à jour de logiciel de dispositif médical (26) authentifiée ;
la réception de journaux, par l'agent de mise à jour de logiciel de dispositif médical en nuage (14), en provenance du premier dispositif médical d'un agent logiciel (20) et d'agents de mise à jour de logiciel (20) des multiples dispositifs médicaux (16) ;
l'identification, par l'agent de mise à jour de logiciel de dispositif médical en nuage (14), de l'incapacité d'un agent donné des agents de mise à jour de logiciel (20) à relayer des fichiers à partir de l'agent de mise à jour de logiciel de dispositif médical en nuage (14) en fonction des journaux (28) reçus ; et
la mise en œuvre, par l'agent de mise à jour de logiciel de dispositif médical en nuage (14), d'une action par rapport à l'agent de mise à jour de logiciel (20) donné en réponse à l'identification de l'incapacité.
